Europäisches Patentamt

European Patent Office

Office européen des brevets

(19)

(11) Publication number: 0 325 018
A2

## (12) EUROPEAN PATENT APPLICATION

(21) Application number: 88301820.2

(22) Date of filing: 02.03.88

(51) Int. Cl.⁴: G01N 33/569 , C07H 21/00 , A61K 31/00

The microorganism(s) has (have) been deposited with American Type Culture Collection under number(s) CRL 9659.

(30) Priority: 23.12.87 US 136978

(43) Date of publication of application: 26.07.89 Bulletin 89/30

(84) Designated Contracting States: AT BE CH DE ES FR GB GR IT LI LU NL SE

(71) Applicant: HEM RESEARCH, INC. 12280 Wilkins Avenue P.O. Box 2245 Rockville, MD 20852(US)

(72) Inventor: Carter, William A. 1 Jaine Lane Birchrunville Pennsylvania(US)

(74) Representative: Votier, Sidney David et al CARPMAELS & RANSFORD 43, Bloomsbury Square London WC1A 2RA(GB)

(54) RNase L inhibitor as a marker for virus infections.

(57) HIV-directed inhibitors of the natural $2'\text{-}5'A$ antiviral defense pathway, diagnostic procedures and materials, and modalities for treating viral disorders are described. HIV-induced inhibitors of RNase L in patients with lymphadenopathy (LAS), AIDS-related complex (ARC) and acquired immune deficiency syndrome (AIDS) were isolated and identified. Functional 2-5A is not able to activate RNase L in the peripheral blood mononuclear cells (PBMC) in these patients; endogenous dsRNA, especially mismatched dsRNA, activates 2-5A synthetase which results in an increased concentration by endogenous 2-5A.

EP 0 325 018 A2

# RNase L INHIBITOR AS A MARKER FOR VIRUS INFECTIONS

## GENERAL BACKGROUND ON AIDS

AIDS (Acquired Immunodeficiency Syndrome) represents a major public health threat of the Twentieth Century. Because of its biologically variable nature, its subtle means of spread from individual to individual, and its "latent" disease period which defies even the ready detection of its very presence, AIDS has rapidly evolved into an epidemiologic dilemma of unprecedented proportions. Indeed, even with the considerable resources already deployed to increase diagnostic capabilities, the available technologies still fall well short of the necessary requirements to gain firm control of this problem within any section of the population, e.g., see Proceedings of Workshop entitled "Experience with HTLV-III Antibody Testing, an Update on Screening, Laboratory and Epidemiological Correlations", sponsored by the Center for Drugs and Biologics, FDA, National Institutes of Health, and Centers for Disease Control, held on July 31, 1985, at the National Institutes of Health, Bethesda, Maryland; see also, Budiansky, S. in Nature, volume 316, page 96, July 11, 1985.

Several different designations for human immunodeficiency virus (HIV) exist; LAV is the designator for the AIDS virus isolated at the Pasteur Institute, Paris, France, and HTLV-III is the designator for the virus isolated at the National Institute of Health, Bethesda, Maryland, U.S.A. Frequently in this text, this virus will be referred to generically or designated HTLV III or LAV or HIV without intending to differentiate between them. Furthermore, the term "HIV" in the specification includes any and all other viruses which may be associated with producing AIDS, whether yet isolated or not. HIV in the singular is meant to include any of the HIV types regardless of their genomic contents. Similarly, AIDS symptoms will be referred to generically to include symptoms caused by the HIV (as defined above), ARC or AIDS related complex, lymphadenapathy syndrome (LAS), and other viruses causing substantially similar clinical conditions.

AIDS follows the progressive deterioration of the immune system caused by infection of human immunodeficiency virus, HIV. Early in the disease, cell mediated immunity becomes impaired through a loss of T (thymus derived) cells which express the so-called T4 antigen, a subset of T cells consisting mainly of T helper and inducer cells. T4 cell deficiency may lead to several deficits in cell-mediated immunity, including deficits in NK (natural killer cell), LAK (lymphokine activated killer cell) and cytolytic T cells. There are also B cell (bone marrow derived) defects in AIDS.

AIDS appears to be a progressive disease, although the rate of transition from one phase to another can be variable. These phases have assigned classifications. Asymptomatic individuals infected by HIV exist (seropositive), as judged by the presence of circulating antibodies against the virus (M.G. Sarngadharan et al, Science, volume 224, p. 506, 1984). If they have no other markers, they are WRI stage patients, by the Walter Reed classification (R.R. Redfield et al, New England Journal of Medicine, volume 314, p. 131 1986). They have been also called pre ARC (ARC = AIDS-related complex), although some of these individuals may never develop ARC symptomatology. Some of these patients may develop lymphadenopathy (WR2) and exhibit a T4 cell deficit (WR3). Next, the ability of lymphocytes to undergo antigen-stimulated proliferation and antigen-stimulated interferon-gamma production decreases and these WR4 patients begin to lose delayed cutaneous hypersensitivity. WR5 patients are usually anergic. They exhibit Herpes Zoster infections, oral candidiasis (thrush), or ARC symptoms which include prolonged fevers, night sweats, fatigue, diarrhea and/or weight loss. This period of ARC is usually also characterized by progressive immune decay. Finally, WR6 individuals experience opportunistic infections constituting full-blown" AIDS, with 50% of the patients dyeing within 12 months (H.W. Murray et al, New England Journal of Medicine, volume 310, p. 883, 1984).

## BACKGROUND OF THE INVENTION

Described are HIV directed inhibitor(s) of the natural $2'-5'$ A antiviral defense pathway. These findings are pertinent to a new diagnostic and/or treatment modality for a variety of viral disorders (e.g., herpes, retrovirus infections, etc.) and/or potentially cancerous conditions as well.

The interferon-induced 2-5A ($2'-5'$-oligoadenylates) synthetase/RNase L system is part of the host defence mechanism against viral infections. Some elements of this system include (i) the dsRNA-dependent 2-5A synthetase, (ii) endogenous 2-5A, and (iii) a 2-5A dependent endoribonuclease (RNase L). A defect in any of these elements of the 2-5A synthetase/RNase L pathways would result in complete or partial abolishment of the antiviral effect.

Elevated levels of 2-5A synthetase have been found in serum and peripheral blood mononuclear cells (PBMC) of humans with some viral infections or humans undergoing interferon therapy. Induction of 2-5A synthetase in PBMC, therefore, seemed to have a potential, though limited, diagnostic value. Moreover, high 2-5A synthetase levels in PBMC indicated an advanced disease state in some viral disorders, but in contrast are a sign of effective interferon therapy of other viral infections. interferon has been found in the serum of AIDS patients, but nonetheless, persistent elevation of 2-5A synthetase was shown for individuals who have progressed from ARC to AIDS. These enormous laboratory/clinical discrepancies could be explained if other elements of the 2-5A synthetase/RNase L system, such as intracellular 2-5A or RNase L, were found to be nonfunctional.. In order to explore this possibility, characterization of the 2-5A synthesized in vivo and RNase L action would be required, as I report in detail herein.

## BRIEF DESCRIPTION OF THE DRAWINGS

The invention is further described with reference to the attached drawings in which:

FIGURE 1 is a photograph of a polyacrylamide gel electrophoresis plate showing the activation of RNase L by 2-5A extracted from PBMC extracts of humans with LAS, ARC or AIDS as determined by ribosomal RNA cleavage assay;

FIGURE 2 is a photograph of a gel electrophoresis plate showing the activation of RNase L level in PBMC extracts as determined by ribosomal RNA cleavage assay;

FIGURE 3 is a photograph of a gel electrophoresis plate showing the restoration of ribosomal RNA cleavage activity in PBMC; and

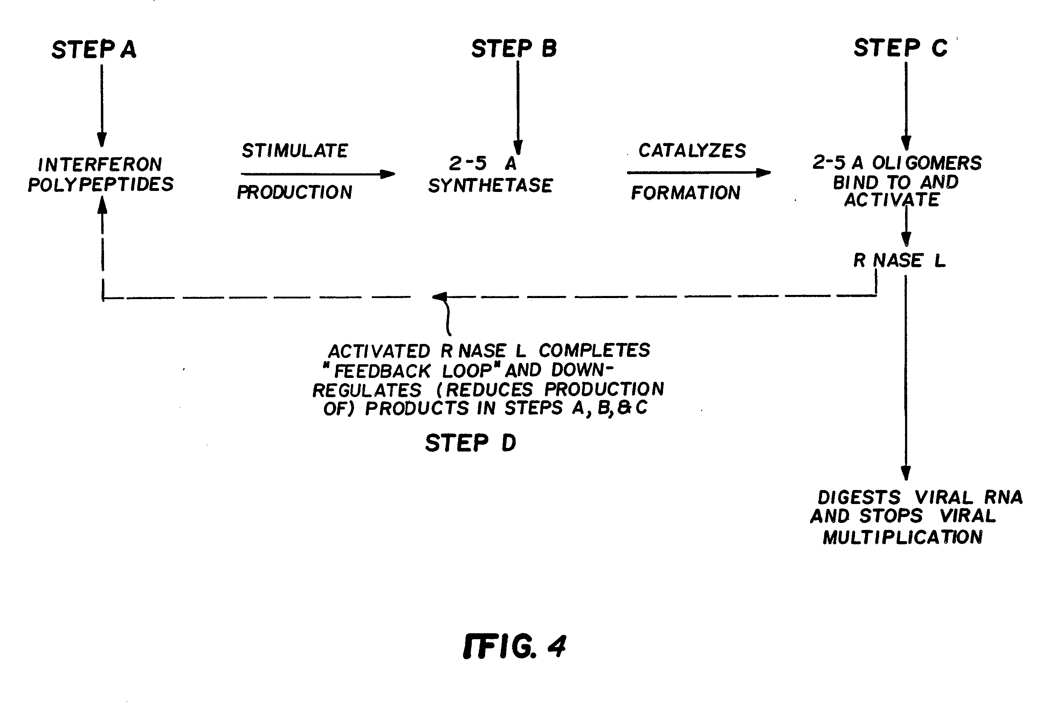

FIGURE 4 is a flow chart illustrating the $2'-5'$ oligadenylate/RNase L pathway.

## DESCRIPTION OF THE INVENTION

An inhibitor substance, released or caused to be released by cells infected with human immunodeficiency viruses, has been discovered. This inhibitor appears to physically attach to the enzyme RNase L, the terminal product of the $2'-5'$ oligoadenylate/RNase L pathway, causing the entire immune system to malfunction and allowing the HIV to multiply uncontrollably. Disclosed are techniques of identifying these inhibitors, either directly or indirectly by the absence of RNase L or inactivity of RNase L or the presence of inhibitors of biochemical intermediates in the $2'-5'$A/RNase L pathway, the inhibitor(s), serving as a marker to detect the presence of HIV or related viruses presenting substantially similar clinical conditions.

Prognostic significance of this marker with respect to the development of full-blown AIDS in patients, particularly those having antibodies to an HIV in their peripheral blood and in tissues including blood, blood fractions, the cells of transplant organs and cellular extracts are also discussed. Procedures for activating RNase L and/or removing or inactivating the RNase L inhibiting substance are described. Therapeutic methods of treating AIDS and related viruses and monitoring the progress of such viruses by activating RNase L and/or removing or inactivating the RNase L inhibitor substance(s) or virus directed to inhibitors are part of this invention. Procedures for preparing antibodies to these viruses, both in vivo and in vitro, using as antigen a specific inhibitor of RNase L or virus directed inhibitors of biochemical intermediates in the $2'-5'$A/RNase L pathway are included in this invention.

Constituents of the 2-5A synthetase/RNase L antiviral pathway were measured in extracts of peripheral blood mononuclear cells (PBMC) from humans with lymphadenopathy (LAS), AIDS-related complex (ARC) and acquired immune deficiency syndrome (AIDS). The level of 2-5A synthetase was shown to be highly elevated in humans with AIDS and at basal level in individuals with ARC or LAS. However, independent of variations in the 2-5A synthetase (2-5A), high endogenous concentrations of 2-5A (up to 35 nM) were detected in humans with LAS, ARC and AIDS. The 2-5A extracted was indeed functional as determined by binding, core-cellulose and ribosomal RNA cleavage assays. Despite the elevated concentrations of endogenous functional 2-5A, no activated RNase L could be demonstrated in any of the humans with LAS, ARC, AIDS. However, RNase L was activated in PBMC of all healthy individuals, even though the concentrations of endogenous 2-5A were less than 5 nM. Mixing experiments provided evidence for the presence of an RNase L inhibitor in five out of five humans with LAS, ARC and AIDS. This inhibitor is transferred and can prevent the 2-5A-induced, RNase L-specific degradation of rRNA in L929 cell extracts. One such inhibitor of RNase L is TCA and ethanol precipitable. The inhibition of RNase L action was abolished by RNase A or protease treatment. My studies suggest that in PBMC of humans with LAS, ARC and AIDS, an endogenous dsRNA activates 2-5A synthetase, which results in an increased concentration of endogenous 2-5A. The ability of the functional 2-5A to activate RNase L is prevented by an HIV-induced inhibitor of RNase L in PBMC of humans with LAS, ARC and AIDS.

I note here the measurement of 2-5A concentration and RNase L function in PBMC extracts by utilizing specific and sensitive assays and demonstrate that a defect in the 2-5A synthetase/RNase L antiviral system in PBMC from humans with LAS, ARC and AIDS lies in the presence of an inhibitor of RNase L but not in the synthesis of nonfunctional 2-5A. The data reported here emphasize that the apparently paradoxical interactions occurring in the virus-infected cell can now be explained in rational, coherent biochemical terms.

## DETAILED DESCRIPTION OF THE DRAWINGS

RNase A bound to acrylic beads (1 mg) or protease bound to carboxymethyl cellulose (1 mg) (Sigma Chemical Co.) were soaked in NP40 lysis buffer (16) (10 $\mu$L) for 2 hours at 30°C, followed by mixing with PBMC extract (100 $\mu$g/assay) in a final volume of 15 $\mu$L and an additional 2 hour incubation at 30°C. During the second incubation, the tubes were gently finger vortexed several times. Control samples were incubated without any enzyme addition. The immobilized enzymes were pelleted by centrifugation (10,000 x g, 5 min, room temperature). Five microliters of the supernatant fractions were added to L929 cell extracts (150 $\mu$g protein/assay) and rRNA cleavage assays were used to measure RNase L-specific activity.

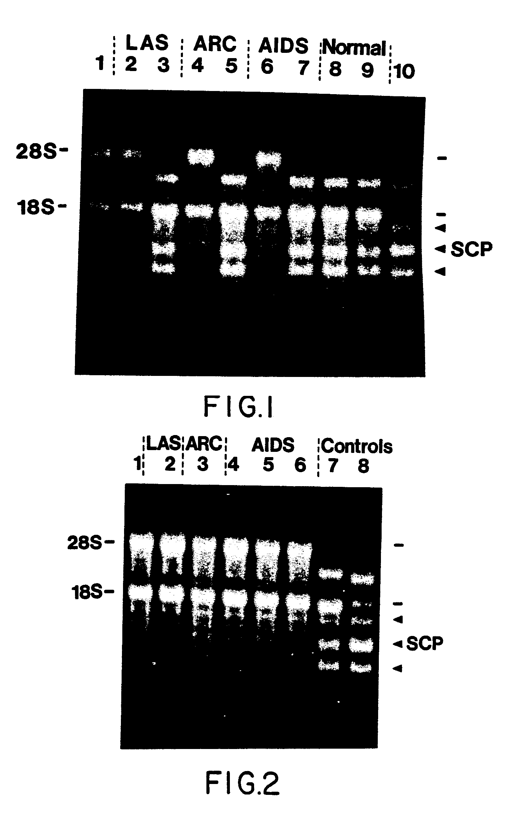

The endogenous 2-5A isolated from PBMC extracts was further characterized by rRNA cleavage assays. The RNase L-generated cleavage patterns with 2-5 A isolated from PBMC were significantly different in L929 cell extracts than those obtained with authentic 2-5A (Fig. 1, compare lanes 3, 5, 7 and 9 with lane 10). For example, the endogenous 2-5A isolated from PBMC activates the RNase L from L929 cells to cleave 28S and 18S rRNA occurs to form three specific cleavage products (Fig. 1, lane 10). An explanation for the differences in the cleavage patterns observed may be that the endogenous 2-5A synthesized in PBMC differs from the authentic 2-5A.

Radiobinding assays have been used to estimate the level of free (not 2-5A-activated) RNase L, a presently-known target enzyme for 2-5A. Additional target enzymes which are equally relevant to my discovery may also be used. PBMC extracts of humans with LAS, ARC and AIDS have 5-7 times lower levels of free RNase L than those of normals (Table 1 column 4). However, the radiobinding assay has limited application in samples where endogenous 2-5A competes with $P_3A_4[^{32}P]pCp$ for binding to RNase L. Consequently, results obtained in radiobinding assays do not accurately reflect levels of RNase L. Therefore, determination of the level of 2-5A-activated RNase L was essential. To achieve this goal, I used a new and sensitive technique described below which permits the measurement of activated RNase L in extracts of PBMC isolated from as little as one milliliter of peripheral blood, a technique based on the cleavage specificity of 2-5A-activated RNase L on rRNA.

Because the rRNA in PBMC is below the level of detection, HL929 cell (an RNase L deficient subclone of the L929 cell line) extracts were substituted as the source of the rRNA. HL929 cells are deposited at the American Type Culture Collection in Rockville, Maryland, USA under accession number      under the terms of hte Budapest Treaty. Using this assay, I can demonstrate that RNase L is not active in PBMC extracts from humans with LAS, ARC and AIDS (Fig. 2, lanes 2-6) where there is a high intracellular accumulation of functional 2-5A. Extracts of PBMC from all healthy individuals tested showed the presence of active RNase L which produced specific cleavage products (Fig. 2, lane 7) even though 2-5A was present at <5 nM (Table 1, column 2).

My observation that biologically active 2-5A was present in PBMC extracts from LAS, ARC and AIDS infected patients raised the question as to why the RNase L was inactive. To determine whether the absence of RNase L activity (as evidenced in Fig. 2, lanes 2-6) was due to either nonfunctional RNase L or due to an inhibitor of RNase L activity, mixing experiments were performed as follows. L929 cell extracts, containing functional RNase L, were mixed with and co-incubated with with PBMC extracts from humans with LAS, ARC and AIDS. No formation of RNase L-specific cleavage products was detectable (Fig. 1, lanes 3, 5 and 7).

It is important to emphasize that the endogenous 2-5A responsible for the activation of RNase L (Fig. 1, lanes 3, 5 and 7) represents at least a four-fold dilution relative to the cell extracts from which these samples were derived (Fig. 1, lanes 2, 4 and 6). These data indicate the presence of a TCA-insoluble RNase L inhibitor(s) in the PBMC of humans with LAS, ARC and AIDS. Such an inhibitor is not found in healthy humans because their PBMC extracts generate RNase L-specific cleavage products following co-incubation with L929 cell extracts (Fig. 1, lane 8). Similarly, the TCA-soluble fraction of PBMC from healthy humans also generates specific cleavage products (Fig. 1, lane 9). The presence of functional 2-5A and the fact that the putative RNase L inhibitor was TCA and ethanol precipitable suggest that the inhibitor of RNase L is not a 2-5A analog as has been recently identified in HSV-1 and retrovirus-infected cells in culture.

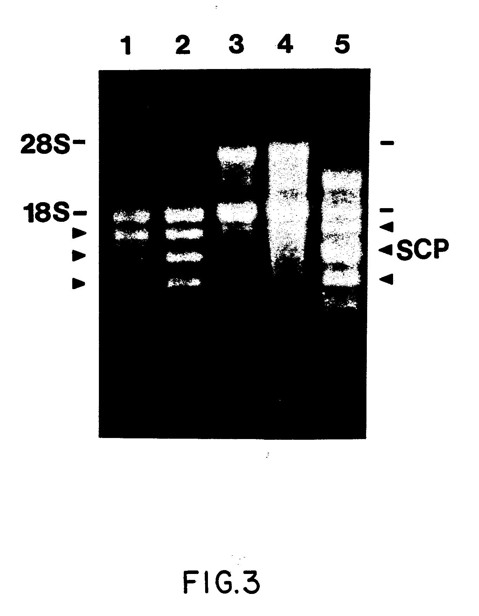

My experiments using degradative enzymes immobilized on solid supports have provided major new insight into the nature of the inhibitor(s) of RNase L found in PBMC extracts from humans with LAS, ARC and AIDS. When PBMC extracts from humans with AIDS were preincubated with immobilized protease or RNase A, and subsequently co-incubated with L929 cell extracts, RNase L activation was restored, as evidenced by the appearance of specific cleavage products (Fig. 3, compare lanes 1 and 2 with lane 3).

My present study demonstrates that a defect in the 2-5A synthetase/RNase L antiviral system in PBMC from humans with LAS, ARC and AIDS can be attributed to an inhibitor of RNase L and not to the lack of functional 2-5A. The RNase L inhibitor is present in PBMC extracts isolated from humans with LAS, ARC and AIDS, which indicates that the inhibitor can exist in all stages of typical retroviral disease development and is also relevant to a variety of other subacute/chronic viral/pathogen infections. Taking into consideration my previous report that all PBMC from humans with LAS, ARC and AIDS tested here has HIV RNA (as determined by molecular hybridization) and HIV infectious centers (as measured by lymphocyte co-cultivation), it is likely that the RNase L inhibitor is virus-induced.

The findings reported here suggest that virus replication modifies the 2-5A synthetase/RNase L system such that the natural antiviral state can not fully be expressed and/or maintained. It is likely that protein(s) or RNA(s) coded by HIV and/or other co-existing retroviruses can inhibit the activation of RNase L.

## Example

Peripheral blood mononuclear cells (PBMC) were isolated on Ficoll-Hypaque; L929 and HL929 (an RNase L deficient subclone of L929) cells were maintained in monolayer culture; and cytoplasmic extracts from L929 and LH929 cells were prepared in glycerol buffer and PMBC extracts were prepared in NP40 lysis buffer, all according to known procedures. Protein concentration of the extracts ranged from 1-15 $\mu g/\mu L$.

Activity of 2-5A synthetase was determined in isolated PBMC extracts (50 $\mu g$ protein/assay) using poly-(rI)-poly(rC)-agarose. 2-5A was isolated from the TCA-soluble fraction of PBMC extracts following Freon/tri-n-octylamine neutralization. The TCA-extracted 2-5A was then lyophilized and redissolved in water such that all samples were standardized in a volume of water that is equivalent to 5 $\mu g$ protein/$\mu L$. Concentrations of 2-5A present in PBMC extracts were measured in radiobinding assays with L929 cell extracts as the source of RNase L. Of the 18,900 dpm of $P_3A_4[^{32}P]pCp$ (specific activity 3000 Ci/mmol, Amersham) added per assay, 9,400 dpm were bound to RNase L in the absence of added 2-5A. Approximately 9-14% of the $P_3A_4[^{32}P]pCp$ was replaced in assays to which 5 $\mu L$ of the isolated 2-5A samples were added. The 2-5A concentration in PBMC was determined by comparison to calibration curves with authentic 2-5A and was based on a calculation that cytoplasmic protein concentration of 25 $\mu g/\mu L$. Concentration of the 2-5A isolated from PBMC was also measured in core-cellulose assays with L929 cell extracts as the source of RNase L. Activation of RNase L by 2-5A in this assay is based on the conversion of poly(U)$[^{32}P]pCp$ to acid soluble fragments. Approximately 5-20% of the total poly(U)$[^{32}P]pCp$ (15,000 dpm) was cleaved in the presence of 2.5 $\mu L$ of the isolated 2-5A samples. Concentrations were determined from calibration curves with authentic 2-5A as described above. Free RNase L level was measured in radiobinding assays after the addition of 20,000 dpm of $P_3A_4[^{32}P]pCp$ to PBMC extracts (50 $\mu g$ protein/assay). The results obtained were as follows

Table 1  Activity of 2-5A synthetase, endogenous 2-5A concentration, free RNase L level and presence of RNase L inhibitor in PBMC extracts from humans with LAS, ARC and AIDS

| PBMC source | 2-5A synthetase[a] | Intracellular-2-5A | | Free RNase L[d] | RNase L activity[e] |
|---|---|---|---|---|---|
| | | Method A[b] | Method B[c] | | |
| column: | 1 (nmole ATP incorporated/mg protein) | 2 (nM) | 3 (nM) | 4 (dpm) | 5 |
| #1 LAS | 30 | 18 | ND[g] | 280 | inhibited |
| #2 ARC | 13 | 25 | 31 | 270 | inhibited |
| #3 AIDS | 195 | 32 | 35 | 270 | inhibited |
| #4 AIDS + KS | 50 | 21 | 15 | 260 | inhibited |
| #5 AIDS | 157 | 24 | 25 | 240 | inhibited |
| Normal[f] | 10 | 5 | ND | 1630 | not inhibited |

[a] standard deviation <9%
[b] determined in radiobinding assays (in duplicate)
[c] determined in core-cellulose assays (in duplicate); standard deviation <1%
[d] determined in radiobinding assays (in duplicate); standard deviation <10%
[e] determined in rRNA cleavage assays; refer also to Figure 1
[f] average of 7 control samples (in duplicate)
[g] ND = not determined

The activity of the interferon-induced enzyme, 2-5A synthetase, in extracts of PBMC obtained from humans with LAS, ARC and AIDS, was measured in in vitro assays and compared to that in extracts from healthy individuals as shown in Table 1, above. The 2-5A synthetase levels in humans with AIDS (#3 and #5) were 16 and 20 times higher than that observed in normal patients (Table 1, column 1). In contrast, the 2-5A synthetase levels in patients with LAS (#1), ARC (#2) and AIDS with Kaposi's sarcoma (KS) (#4) were only elevated 1.3 to 5 times supporting baseline observations made by others.

EP 0 325 018 A2

To determine whether this in vitro measure of 2-5A synthetase was an indication of functional enzyme in vivo 2-5A was extracted from the PBMC. The biological activities of the 2-5A from the TCA-soluble fraction of PBMC extracts were extensively characterized by radiobinding and core-cellulose assays. Concentrations of 2-5A were similar as determined by the two assay procedures (Table 1, columns 2 and 3). The agreement between results obtained by methods A and B (Table 1, columns 2 and 3) demonstrates that the 2-5A isolated from human PBMC extracts can bind to and activate RNase L from L929 cells to the same extent as authentic 2-5A.

The apparent absence of quantitative correlation between the levels of 2-5A synthetase and endogenous 2-5A in LAS, ARC and AIDS (Table 1, compare column 1 with column 2 and 3) can now be explained, for the first time, as follows. Determination of 2-5A synthetase in in vitro assays is based on the partial purification via poly(rI)-poly(rC)-agarcse; consequently, this assay measured only the free 2-5A synthetase and not the 2-5A synthetase that is activated in vivo by dsRNA. However, direct measurement of endogenous 2-5A synthetase plus the 2-5A degradative enzymes (i.e., $2'$-phosphodiesterase and phosphatases). Therefore, the accumulation of 2-5A in PBMC of humans with LAS, ARC and AIDS indicates a low activity of the 2-5A degradative enzymes. The presence of 2-5A in PBMC also indicates that there is an endogenous dsRNA hitherto undetected in both normal subjects and AIDS patients.

Mismatched double-stranded RNA is a known form of macromolecular RNA (see U.S. Patent No. 4,024,222 and U.S. Patent No. 4,130,641) in which destabilization of the double helix prevents base pairing. Mismatched dsRNA is well known for its interferon-induction properties which indicate a mechanism unrelated to interferon induction per se (e.g., European Patent Application 83305426.5 filed August 15, 1983, entitled "Antiproliferative Action of dsRNAs on Tumor Cells") (EP-A-0 113 162).

A typical therapeutic embodiment of mismatched double-stranded RNA is the synthetic dsRNA formed by complexes of polyriboinosinic and polyribocytidylic/uridylic acid, such as $rI_n \, r(C_x, U \text{ or } G)_n$ where x has a value from 4 to 29, e.g., $rI_n.r(C_{12}U)_n$ herein referred to as AMPLIGEN®, a registered U.S. trademark of HEM Research, Inc., of Rockville, Maryland, USA. Many mismatched dsRNA polymers which behave similarly to Ampligen have been studied. The key therapeutic advantage of mismatched dsRNAs over other forms of natural and/or synthetic dsRNAs is their reduced toxicity in animals and man. For example, Lampson et al in U.S. Patent No. 3,666,646 described earlier complexes of dsRNA which are capable of triggering various interferon-related effects, but the toxicity of such compounds precluded any clinical utility in the treatment of cancer or related disorders.

Mismatched dsRNA, e.g., Ampligen, is now known to have therapeutic activity against certain human tumors, particularly kidney cancer. Although currently thought of as purely an "interferon inducer", dsRNAs are active on certain human tumors which are completely resistant to interferon itself as fully described in European Patent Application 83305426.5 (EP-A-0 113 162) of which the present applicant is an inventor.

In another patent application (see European Patent Application 86306582.7, filed August 26, 1986 entitled "Modulation of Virus-Related Events by Double-Stranded RNAS),(EP-A-0 213 921) the inventor describes the inhibition of AIDS virus in human cell culture by dsRNAs using Ampligen as a prototype dsRNA.

By "matched dsRNA" are meant those in which hydrogen bonding (based stacking) between the counterpart strands is relatively intact, i.e., is interrupted on average less than one base pair in every 29 consecutive base residues. The term mis-matched dsRNA" should be understood accordingly.

The dsRNA may be a complex of a polyinosinate and a polycytidylate containing a proportion of uracil bases or guanidine bases, e.g., from 1 in 5 to 1 in 30 such bases (poly I. poly (C4-29 x > U or G).

The dsRNA may be of the general formula $rI_n.(C_{12}U)_n$. Other suitable examples of dsRNA are discussed below.

The mismatched dsRNAs preferred for use in the present invention are based on copolynucleotides selected from poly $(C_n, U)$ and poly $(C_n, G)$ in which n is an integer having a value of from 4 to 29 and are mismatched analogs of complexes of polyriboinosinic and polyribocytidilic acids, formed by modifying $rI_n.rC_n$ to incorporate unpaired bases (uracyl or guanine) along the polyribocytidylate $(rC_n)$ strand. Alternatively, the dsRNA may be derived from poly (I). poly(C) dsRNA by modifying the ribosyl backbone of polyriboinosinic acid $(rI_n)$ e.g., by including $2'$-O-methyl ribosyl residues. These mismatched analogs of $rI_n.rC_n$, preferred ones of which are of the general formula $rI_n.r(C_{12},U)_n$, are described by Carter and Ts'o in U.S. Patents 4,130,641 and 4,024,222 the disclosures of which are hereby incorporated by reference. The dsRNA's described therein generally are suitable for use according to the present invention.

Specific examples of mismatched dsRNA for use in the invention include: -

poly (I). poly $(C_4, U)$

poly (I). poly $(C_7, U)$

7

poly (I). poly (C$_{13}$, U)
poly (I). poly (C$_{22}$, U)
poly (I). poly (C$_{20}$, G)
poly (I). poly (C$_{29}$, G) and
poly (I). poly (C$_p$) 23 G>p

Pharmaceutical compositions contemplated by this invention include those adapted for parenteral administration in a suitable pharmaceutical vehicle. Thus, for example, parenteral solutions, suspensions and dispersions can be prepared as required according to known pharmaceutical techniques with sterile or pyrogen-free water as the solvent/diluent optionally also with physiologically acceptable salts.

The amount of mismatched dsRNA administered is preferably sufficient to achieve a peak blood concentration of from 0.01 micrograms per milliliter of the dsRNA level through the body fluid up to 1000 micrograms per milliliter in the systemic blood circulation immediately following administration at a point distal from the site of infusion.

Alternatively, an effective concentration may be added to the blood product immediately prior to injection into the recipient. In such cases, the operator may simply refer to a standard table of body fluid volumes which interrelate the weight of the recipient to his or her body fluid volume, which is the total of the body fluid volume of the patient and the body fluid volume available for equilibration with the necessary quantity of the dsRNA. A sixty to seventy kilogram patient will have a body fluid volume of approximately five to six liters.

## Claims

1. A method for determining the presence of HIV or another virus causing substantially similar clinical conditions, comprising determining the presence of an RNase L inhibitor, a marker for the presence of HIV, or genetic information directed by HIV, in an animal's cellular extract.

2. The method of claim 1, for determining the presence of occult human immunodeficiency viruses in a human comprising detecting the presence of an RNase L inhibitor in human biological fluid or cells.

3. The method of claim 2, in which the biological fluid is blood or a blood fraction.

4. The method of claim 2, in which the cells are from a skin graft or a transplantable human organ.

5. The method of any one of claims 1 to 4 for determining the projected incidence of HIV in an animal comprising examining an extract of the animal's tissue and determining the presence or absence of at least one Rnase L inhibitor, the presence positively indicating the projected presence of HIV.

6. dsRNA for use in treating an animal, determined by the method of any one of claims 1 to 5 to have the presence of HIV, an occult human immunodeficiency virus or the projected incidence of HIV, by administration of an amount of said dsRNA sufficient to reduce the amount of RNase L inhibitor(s) of HIV-induced conditions or other such viruses which cause similar biochemical and/or clinical conditions, and/or for continuing administration until the patient's clinical symptoms are stabilised or improved.

7. An HIV-specific inhibitor of RNase L or a virus directed inhibitor of biochemical intermediates in the 2' - 5' A/RNase L pathway, for administration as an antigen to an animal for conferring immunity to HIV or for treating an HIV infection.

8. The inhibitor of claim 7, which is dsRNA.

9. The dsRNA of claim 8, which is a mismatched dsRNA.

10. The dsRNA of claim 8 or claim 9, in which the dsRNA is a complex of a polyinosinate and a polycytidilate containing from 1 in 5 to 1 in 30 uracil or guanidine bases, and preferably of the general formula $rl_n(C_{12}U)_n$.

11. Use of the inhibitor as defined in any one of claims 7 to 10 in the production of a medicament for use in any one of the medical treatments defined in either of claims 6 and 7.

FIG.I

FIG.2

FIG.3

**STEP A**

INTERFERON
POLYPEPTIDES

STIMULATE
——————→
PRODUCTION

**STEP B**

2-5 A
SYNTHETASE

CATALYZES
——————→
FORMATION

**STEP C**

2-5 A OLIGOMERS
BIND TO AND
ACTIVATE

R NASE L

ACTIVATED R NASE L COMPLETES
"FEEDBACK LOOP" AND DOWN-
REGULATES (REDUCES PRODUCTION
OF) PRODUCTS IN STEPS A, B, & C

**STEP D**

DIGESTS VIRAL RNA
AND STOPS VIRAL
MULTIPLICATION

**ΓFIG. 4**

EP 0 325 018 A2